# EUROPÄISCHE PATENTSCHRIFT

(11) **EP 2 862 530 B1**
(45) Veröffentlichungstag und Bekanntmachung des Hinweises auf die Patenterteilung: **03.10.2018**
(21) Anmeldenummer: 13189566.6
(22) Anmeldetag: 21.10.2013
(51) Int. Cl.: A61B 18/04

(54) **Instrumententestanordnung mit Testeinrichtung**
Instrument test assembly with a test device
Agencement de test d'instruments comprenant un dispositif de test

(43) Veröffentlichungstag der Anmeldung: 22.04.2015
(73) Patentinhaber: Erbe Elektromedizin GmbH, 72072 Tübingen (DE)
(72) Erfinder: Nold, Bernhard Tobias, 72070 Tübingen (DE); Zenker, Mathias, 72074 Tübingen (DE); Selig, Peter, 72379 Hechingen (DE)
(74) Vertreter: Rüger Abel

(56) Entgegenhaltungen:
- EP-A1- 1 693 014
- EP-A1- 1 764 057
- EP-A1- 2 537 479
- DE-A1- 19 839 826

## Beschreibung

Die Erfindung betrifft eine Instrumententestanordnung insbesondere für medizinische Instrumente zur Argon-Plasma-Koagulation.

Argonplasma-Koagulationsinstrumente nutzen zur Erzeugung eines Argonplasmas einen Funken, der aus einem HF-Generator gespeist wird. Vor der Durchführung der eigentlichen Behandlungen führen Anwender gerne einen kurzen Test durch, um zu sehen ob das Instrument arbeitsfähig ist. So können beschädigte Instrumente und falsche Einstellungen frühzeitig erkannt und der Einsatz solcher Instrumente am Patienten vermieden werden. Bei Sonden oder Instrumenten zur Argon-Plasma-Koagulation kann die Zündung des Funkens beeinträchtigt sein, wenn die letzte Argongas-Spülung zeitlich länger zurückliegt oder wenn eine schon einmal benutzte, wieder verwendbare Sonde zum Einsatz kommt. Anwender, die in der Vergangenheit mit schlechtem Zündverhalten solcher Sonden konfrontiert waren, neigen dazu, die Funktionsfähigkeit, insbesondere die Zündfähigkeit, der Sonde vor deren Einsatz zu überprüfen. Diese Vorgehensweise wird häufig auch von Herstellern von Sonden zur Argonplasma-Koagulation empfohlen, um insbesondere bei endoskopischen Sonden sicherzustellen, dass diese nur dann in das Endoskop eingeführt bzw. am Patienten eingesetzt werden, wenn die Sonde bzw. das Instrument einwandfrei funktioniert sowie wenn am Gerät passende Einstellungen gewählt wurden.

Eine Sonde kann nicht einfach am Patienten ausprobiert werden, weil dadurch Verletzungen entstehen können. Der Zündtest an undefiniert geerdeten Metallteilen ist unzulässig, weil er zur Verletzung von Patient und Personal sowie zur Beschädigung elektrischer und elektronischer Einrichtungen führen kann. Dennoch kann nicht ausgeschlossen werden, dass Anwender solche unsachgemäßen Tests vornehmen.

Aus der Praxis sind vom Inhaber des vorliegenden Schutzrechts auf den Markt gebrachte Zündtestadapter bekannt. Ein solcher ist anstelle der Neutralelektrode in die Neutralbuchse des speisenden Geräts einzustecken und er weist eine definiert geerdete Elektrode auf. Daher ist ein Umstecken am Gerät erforderlich. Das kann dazu führen, dass der Zündtestadapter nicht verwendet wird und der Anwender entweder keinen Zündtest durchführt oder doch wieder undefiniert geerdete Punkte (Gerätewagen, Infusionsständer) für den Zündtest verwendet.

Aus der EP 1 764 057 A1 ist ein Adapterstecker bekannt, der zwischen den Neutralleiteranschluss des Generators und den Neutralstecker der Patientenelektrode in den elektrischen Kreis einzufügen ist. Dieser Zündtestadapter weist einen leitfähigen Bereich auf, an dem das plasmachirurgische Instrument auf Funktion getestet werden kann. Zwischen dem leitfähigen Bereich und dem Neutralleiter des Steckers kann ein Widerstand vorgesehen sein.

Davon ausgehend ist es Aufgabe der Erfindung, ein Konzept anzugeben, mit dem sich auf komfortable Weise die Funktionsfähigkeit, insbesondere die Zündfähigkeit, eines Instruments oder einer Sonde vor dem Einsatz am Patienten testen lässt.

Dazu sieht die Erfindung eine Instrumententestanordnung vor, mit der die Sonde oder das medizinische Instrument unter definierten Bedingungen gefahrlos überprüfbar ist. Die Instrumententestanordnung kann als modifiziertes Neutralleiterkabel, als Zwischenstecker oder an dem speisenden Gerät selbst ausgebildet sein.

Zu dem Neutralleiterkabel gehört ein Gerätestecker für den Neutralanschluss des medizinischen Geräts. Von dem Gerätestecker erstreckt sich das flexible Neutralleiterkabel zu einem Neutralelektrodenanschluss oder einer Neutralelektrodenanordnung. In einer Ausführungsform ist die Instrumententestanordnung an dem Neutralleiterkabel angeordnet. Zu der Instrumententestanordnung gehört ein Zündtestelektrodenträger, der mindestens zwei Zündtestelektroden aufweist, die elektrisch mit dem Leiter des Kabels verbunden ist.

Der Zündtestelektrodenträger kann an den Gerätestecker angebaut, in den Gerätestecker eingebaut, als Teil des Gerätesteckers ausgebildet, im Verlaufe des flexiblen Kabelabschnitts oder auch an dem Neutralelektrodenanschluss oder an der Neutralelektrodenanordnung angeordnet sein. Der Zündtestelektrodenträger enthält mindestens zwei Zündtestelektroden, die elektrisch mit dem mindestens einen Leiter des flexiblen Kabelabschnitts verbunden sind.

Ist die Instrumententestanordnung als Zwischenstecker ausgeführt, hat sie geräteseitig einen dort einzusteckenden Stecker und kabelseitig eine Steckbuchse. Zwischen beiden kann ein starres Gehäuse oder ein Kabelabschnitt vorgesehen sein. In letzterem Fall bildet der Zwischenstecker ein Neutralleiter-Verlängerungskabel. Die Zündtestelektroden können an jeder geeigneten Stelle des Zwischensteckers angeordnet sein.

Die Splitstufe hat die Aufgabe, das korrekte Anlegen der Teilelektroden an einen Patienten durch Widerstands- oder Impedanzmessung zu überwachen. Die Instrumententestanordnung hat zwei Zündtestelektroden, diese können hinter den beiden Neutralleitern angeschlossen sein, die zu der zwei- oder mehrpoligen Neutralbuchse und von dort über das Neutralleiterkabel zu der Neutralelektrodenanordnung führen.

Bei allen oben genannten Konzepten wird sichergestellt, dass der Stromkreis des testweise gezündeten elektrischen Funkens nicht den Patienten einschließt, so dass Beeinträchtigungen des Patienten ausgeschlossen sind. Der Test kann außerdem durchgeführt werden, ohne dass die Neutralelektrode von dem Patienten oder der Gerätestecker von dem Gerät entfernt werden müsste. Die erfindungsgemäße Instrumententestanordnung gestattet somit den Funktionstest auch in geschäftiger Umgebung mit minimalem Zeitaufwand.

Das Neutralleiterkabel weist zwei (oder mehrere) Neutralleiter auf, die sich von dem Gerät oder Stecker zu dem Neutralelektrodenanschluss oder der Neutralelektrodenanordnung erstrecken. Es wird mit einer in mehrere (zumindest zwei) Teilelektroden aufgeteilten Neutralelektrode gearbeitet. Die korrekte Anlage der beiden Teilelektroden an dem Patienten lässt sich durch Messung der Impedanz oder des Widerstands zwischen den beiden Teilelektroden überprüfen. Vorzugsweise enthält das speisende Gerät eine entsprechende Prüfschaltung, die den Widerstand zwischen den beiden Leitern und somit zwischen den beiden an den Patienten angelegten Teilelektroden misst. Der Zündtestelektrodenträger weist bei einem solchen Konzept für jeden Leiter jeweils mindestens eine Zündtestelektrode auf, die elektrisch mit dem Leiter verbunden ist. Vorzugsweise ist die Verbindung zwischen der Zündtestelektrode und dem Leiter über ein Bauelement mit einer elektrischen Impedanz vorzugsweise mit resistiver oder kapazitiver Charakteristik hergestellt. Auf diese Weise wird vermieden, dass der testweise gezündete Funke die beiden Leiter miteinander kurzschließt oder nur einen der Leiter kontaktiert. In beiden Fällen könnte es sonst zu einem Ansprechen eines Überwachungssystems kommen, das die korrekte Anlage der Teilelektroden der Neutralelektrodenanordnung überprüft.

Der Umfang der Erfindung wird durch den anhängenden Anspruch 1 festgelegt. Weitere Einzelheiten vorteilhafter Ausführungsformen der Erfindung sind Gegenstand von Ansprüchen der Beschreibung oder der Zeichnung.

Es zeigen:
Figur 1 ein medizinisches Gerät zur Speisung eines HF-Chirurgischen Instruments sowie ein Neutralleiterkabel zur Verbindung einer Neutralleiteranordnung mit dem Gerät in schematischer Darstellung,
Figur 2 das Gerät und das Neutralleiterkabel mit Instrumententestanordnung in schematisierter Darstellung,
Figuren 3 bis 8 abgewandelte Ausführungsformen der erfindungsgemäßen Instrumententestanordnung in schematischer Darstellung,
Figur 9 einen Gerätestecker als Teil einer Instrumententestanordnung in schematischer Seitenansicht und
Figuren 10 bis 14 verschiedene Ausführungsformen von Instrumententestanordnungen jeweils in schematischer Darstellung.

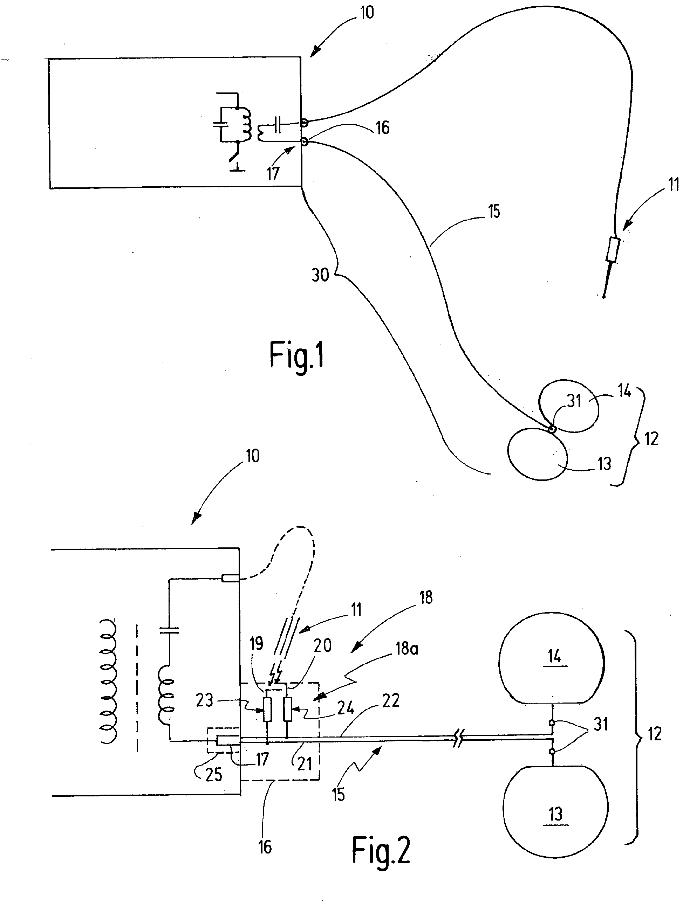

In Figur 1 ist ein medizinisches Gerät 10 zur Speisung eines elektrochirurgischen Instruments 11 veranschaulicht, das auf biologisches Gewebe einwirkt. Bei dem Instrument 11 kann es sich um ein offenchirurgisch, laparoskopisch oder auch ein endoskopisch zu nutzendes Instrument, eine sogenannte Sonde handeln. Insbesondere kann das Instrument 11 eine Argonplasma-Koagulationssonde sein.

Der mit dem Instrument 11 in das biologische Gewebe eingeleitete Strom wird über eine Neutralelektrodenanordnung 12 zu dem Gerät 10 zurückgeführt. Die Neutralelektrodenanordnung 12 umfasst zwei oder mehrere Teilelektroden 13, 14, die zu einem Neutralleiterkabel 15 gehören oder an dieses angeschlossen sind. Das Neutralleiterkabel 15 stellt eine flexible Verbindung zwischen der Neutralelektrodenanordnung 12 und dem Gerät 10 her. An seinem geräteseitigen Ende weist das Neutralleiterkabel 15 einen Gerätestecker 16 auf, der in die Neutralbuchse 17 des Gerätes 10 eingesteckt ist.

Wie Figur 2 zeigt, kann das Neutralleiterkabel 15 mit einer Instrumententestanordnung 18 versehen sein, die dazu dient, die Zünd- und Funktionsfähigkeit des Instruments 11 prüfen zu können.

Die Instrumententestanordnung 18 umfasst zwei Zündtestelektroden 19, 20, die elektrisch mit Leitern 21, 22 des Neutralleiterkabels 15 verbunden sind. Die Zündtestelektrode 19 ist mit dem Leiter 21 vorzugsweise über ein Impedanzbauelement, beispielsweise ein resistives, vorzugsweise ein ohmsches Bauelement 23 verbunden. Der Widerstandswert kann beispielsweise 20 Ohm betragen. Vorzugsweise liegt er im Bereich zwischen 10 und 50 Ohm. Die Zündtestelektrode 20 ist vorzugsweise ebenfalls über ein entsprechendes Impedanzbauelement 24 mit dem Leiter 22 verbunden. Sein Widerstandswert liegt wiederum vorzugsweise im Bereich zwischen 10 Ohm und 50 Ohm, beispielsweise bei 20 Ohm. Die beiden Impedanzbauelemente 23, 24 können gleich oder auch leicht unterschiedlich ausgebildet sein.

Der Leiter 21 ist mit der ersten Teilelektrode 13 verbunden. Der Leiter 22 ist mit der zweiten Teilelektrode 14 verbunden. Eine Splitschaltung 25 ist in Figur 2 lediglich schematisch angedeutet und dient dazu, den ohmschen Widerstand oder die Impedanz zwischen den beiden Teilelektroden 13, 14 zu messen. Auf diese Weise wird erfasst, ob beide Teilelektroden 13, 14, und somit die gesamte Neutralelektrodenanordnung 12, einen ausreichenden elektrischen Kontakt zum Patienten aufweist.

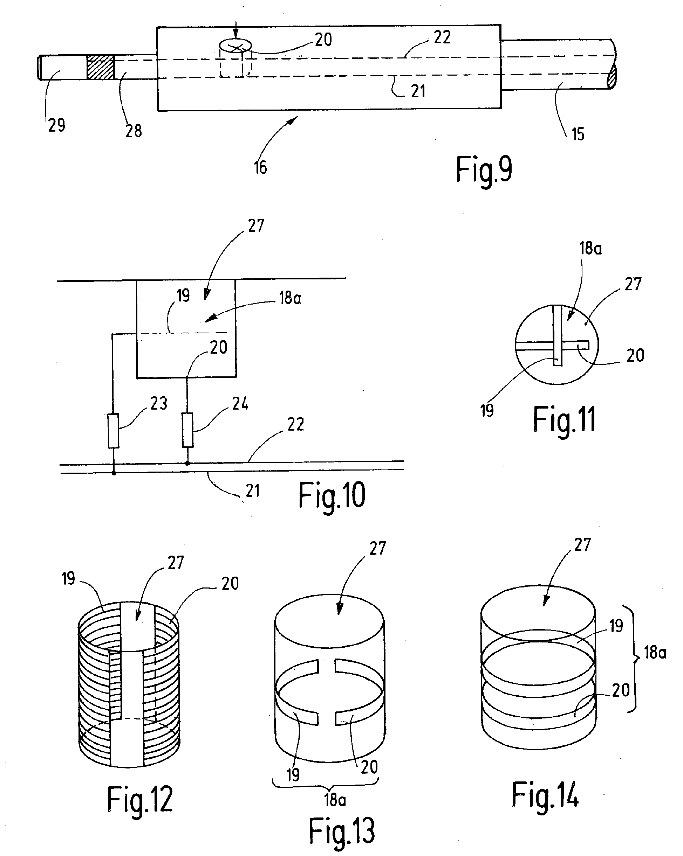

Die Instrumententestanordnung 18 mit den beiden Zündtestelektroden 19, 20 kann im Gerätestecker 16 angeordnet sein, der in Figur 2 lediglich schematisch gestrichelt angedeutet ist. Etwas körperlicher ist er in Figur 9 dargestellt, wobei die bereits eingeführten Bezugszeichen vorausgesetzt und genutzt werden. Der Gerätestecker 16 kann aus einem Kunststoffmaterial oder einem sonstigen isolierenden Material bestehen, das einen Isolatorkörper 26 bildet. Der Gerätestecker 16 kann auch ein Metallgehäuse aufweisen, in das ein entsprechender Isolatorkörper eingesetzt ist. In dem Isolatorkörper 26 kann eine Vertiefung 27 ausgebildet sein, in der die beiden Zündtestelektroden 19, 20 angeordnet sind. Die Zündtestelektroden 19, 20 können, wie beispielsweise Figur 11 zeigt, durch zwei sich in geringem Abstand überkreuzenden Metallnadeln oder Drähte gebildet sein. Auf die Darstellung der Impedanzbauelemente 23, 24 wurde in Figur 9 verzichtet. Erkennbar ist aber, dass die beiden Leiter 21, 22 an verschiedene Kontakte 28, 29 eines von dem Gerätestecker 16 wegragenden Anschlussstiftes geführt sind. Der Anschlussstift wird bei Verwendung in die Neutralelektroden-Buchse des Geräts gesteckt.

Das Gerät 10 und das Instrument 11 sowie insbesondere die Instrumententestanordnung 18 arbeiten wie folgt:
Um eine Behandlung mit dem Instrument 11 durchzuführen, wird dieses zunächst wie in Figur 1 dargestellt mit dem Gerät 10 verbunden. Außerdem wird die Neutralelektrodenanordnung 12 an dem menschlichen oder tierischen Patienten angebracht. Der Anwender wird dann die Funktions- und insbesondere die Zündfähigkeit des Instruments 11 überprüfen wollen. Dazu bringt er das distale Ende der Sonde oder des Instruments 11 in die Nähe der Vertiefung 27 und aktiviert dann den Generator des Geräts 10. Bei zündfähigem Instrument 11 springt ein Funke zu den Zündtestelektroden 19, 20, wobei der so testweise fließende Strom über beide Leiter 21, 22 zu dem Gerät 10 abfließt. Die Neutralelektrodenanordnung 12 verbleibt auf Massepotenzial. Wegen der gleichmäßigen Stromaufteilung erkennt die Splitschaltung 25 kein Fehlersignal. Sie sieht die gleichen Verhältnisse wie bei einem Eingriff oder einer Behandlung am Patienten.

Die Instrumententestanordnung 18 ist im vorliegenden Ausführungsbeispiel Teil des Gerätesteckers 16 und somit Teil eines Neutralleiteranschlusskabels 30. Zu diesem gehören der Gerätestecker 16, das Neutralleiterkabel 15 sowie die Neutralelektrodenanordnung 12. Alternativ kann anstelle der Neutralelektrodenanordnung 12 auch lediglich ein Neutralelektrodenanschluss 31 zu dem Neutralleiteranschlusskabel 30 gehören, an dem eine oder mehrere Neutralelektroden anzuschließen sind.

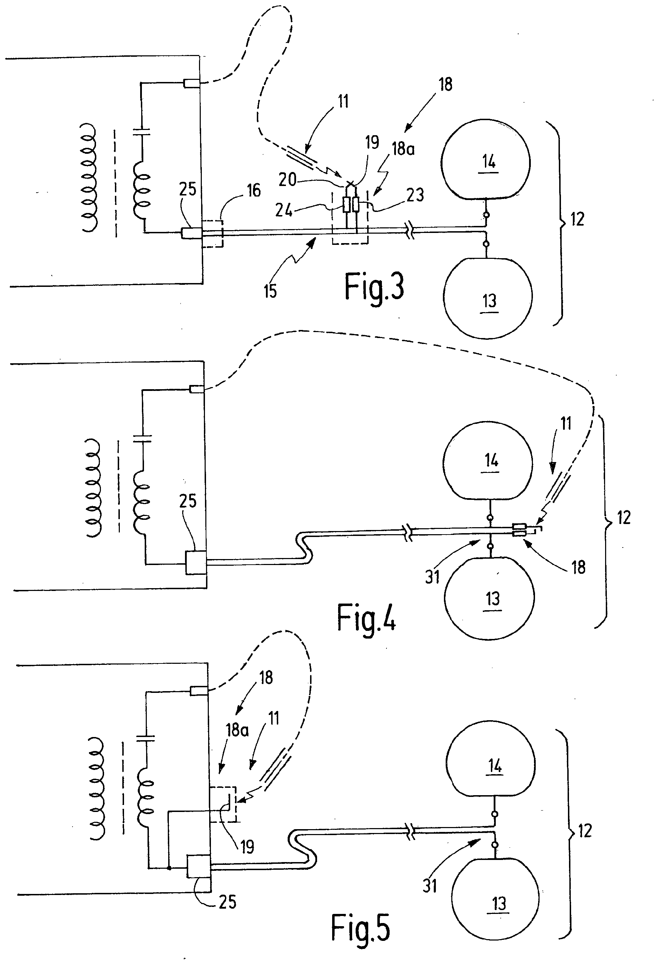

Die Instrumententestanordnung 18 muss nicht zwingend an dem Gerätestecker 16 angeordnet sein. Sie kann dennoch Teil des Neutralleiteranschlusskabels 30 sein. Ein Ausführungsbeispiel dazu ergibt sich aus Figur 3. Wie ersichtlich kann die Instrumententestanordnung 18 im Verlauf des Neutralleiterkabels 15 zwischen dem Gerätestecker 16 und der Neutralelektrodenanordnung 12 angeordnet sein. Die Instrumententestanordnung 18 kann dazu ein eigenes nicht weiter veranschaulichtes isolierendes Gehäuse aufweisen, in oder an dem die Zündtestelektroden 19, 20 angeordnet sind. Eine abgewandelte Instrumententestanordnung gemäß Figur 3 kann auch eine Anschlussmöglichkeit für das Neutralelektrodenkabel 15 aufweisen, so dass das Neutralelektrodenkabel 15 zweiteilig ausgebildet ist. Ein erster Teil des Neutralelektrodenkabels 15 der Bestandteil der Instrumententestanordnung 18 ist, führt von dieser an das Gerät 10. Ein zweiter Teil des Neutralelektrodenkabels 15 ist mit der Instrumententestanordnung 18 lösbar beispielsweise mittels einer Steckverbindung verbunden und führt von dieser zur Neutralelektrode.

Wie Figur 4 zeigt kann die Instrumententestanordnung 18 auch an der Neutralelektrodenanordnung 12, zum Beispiel an dem Neutralelektrodenanschluss 31 angeordnet sein. Wiederum kann die Instrumententestanordnung 18 ein eigenes Isolatorgehäuse aufweisen oder zum Beispiel in dem Neutralelektrodenanschluss 31 angeordnet sein.

Bei einer abgewandelten Ausführungsform gemäß Figur 5 ist die Instrumententestanordnung 18 als Teil des Geräts 10 ausgebildet. Die Zündtestelektrode 19 ist vor der Splitschaltung 25 an den Neutralleiter angeschlossen. In diesem Fall genügt eine einzige Zündtestelektrode, die in einem Isolatorgehäuse in einer Ausnehmung angeordnet ist.

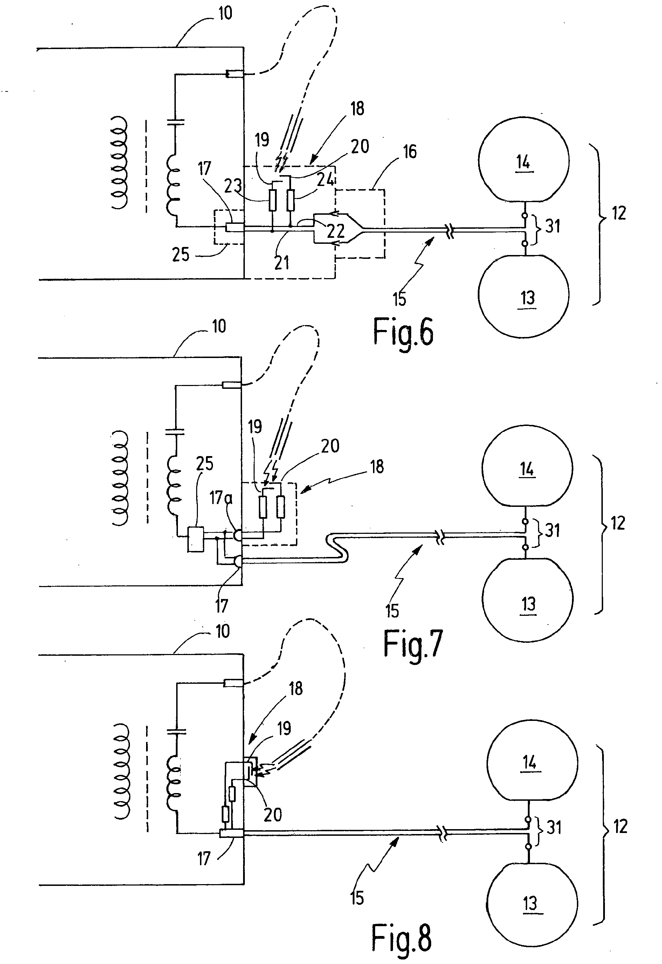

Bei einer weiter abgewandelten Ausführungsform gemäß Figur 6 ist die Instrumententestanordnung 18 als Zwischenstecker ausgebildet, der zwischen dem Gerätestecker 16 und der Neutralbuchse 17 angeordnet ist. Er kann ein eigenes Isolierstoffgehäuse aufweisen, in dem wiederum die Zündtestelektroden 19, 20 und die Impedanzbauelemente 23, 24 angeordnet sind. Die Leiter 21, 22 führen zu einer an dem Zwischenstecker vorgesehenen Buchsenanordnung, in die der Gerätestecker 16 einzuführen ist. Ansonsten gilt die vorige Beschreibung entsprechend.

Die Instrumententestanordnung 18 kann auch an einer gesonderten Buchse 17a angeordnet sein, die gemäß Figur 7 an dem Gerät 10 vorgesehen und elektrisch parallel zu der Buchse 17 geschaltet ist. In diese gesonderte Buchse 17a kann wiederum die Instrumententestanordnung 18 eingesteckt werden, die mit den beiden Testelektroden 19, 20 dem Anwender eine Möglichkeit zum Ausprobieren einer Sonde oder seines Instruments bietet. Auch hierzu gilt die vorige Beschreibung ergänzend entsprechend.

Eine weitere Ausführungsform ergibt sich aus Figur 8. Die Instrumententestanordnung 18 ist dort ähnlich Figur 5 in das Gerät 10 eingebaut. Jedoch sind zwei Zündtestelektroden 19, 20 vorgesehen, die in oder an dem Gerät 10 angeordnet sind. Das Gerät 10 kann dazu einen entsprechenden Isolierstoffkörper aufweisen, der die beiden Zündtestelektroden 19, 20 beherbergt, die zum Beispiel wiederum in einer Vertiefung frei liegen. Die vorige Beschreibung gilt ergänzend.

Zur Beschreibung ergänzend wird auf die Figuren 11 bis 14 verwiesen. Anders als in Figur 11 dargestellt können die Zündtestelektroden 19, 20 eine Vielfalt verschiedener Formen aufweisen. Beispielsweise können in der Vertiefung 27 angeordnete Zündtestelektroden 19, 20 gemäß Figur 10 unterschiedliche Bauformen haben. Die Zündtestelektrode 19 kann als Draht oder Spitze oder als Sieb bzw. Gitter ausgebildet sein, unterhalb dessen die Zündtestelektrode 20 als Sieb, Gitter, Draht, leitende Fläche oder leitender Becher angeordnet ist.

Alternativ ist es möglich, die Zündtestelektroden 19, 20 an der Wandung einer Vertiefung 27 anzubringen. Figur 12 veranschaulicht dazu eine Ausführungsform, bei der die Zündtestelektroden 19, 20 schalenförmig am Umfang der zylindrischen Vertiefung 27 angeordnet sind.

Alternativ können die Zündtestelektroden 19, 20, wie es Figur 13 zeigt, an der inneren Wandung der Vertiefung 27 als schmale bogenförmige Streifen angeordnet sein, die wiederum in Umfangsrichtung voneinander distanziert sind.

Es ist auch möglich, die Zündtestelektroden 19, 20, wie es Figur 14 zeigt, als axial distanzierte Ringe auszubilden.

Auch weitere Kombinationen vorhandener vorgestellter Elektrodenformen sind möglich, beispielsweise die Anordnung einer ringförmigen Zündtestelektrode in Verbindung mit einer Nadel oder stabförmigen Zündtestelektrode nach Figur 11.

Um dem Anwender eines HF-chirurgischen Instruments 11 einen unschädlichen kurzen Testbetrieb zu ermöglichen, beispielsweise um die Zündfähigkeit einer Argonplasmasonde zu testen, ist eine Instrumententestanordnung 18 vorgesehen, die in ein speisendes medizinisches Gerät 10 oder ein Neutralleiteranschlusskabel 30 eingebaut ist oder die als Zwischenstecker bereitgestellt wird, der zwischen das Gerät 10 und das Neutralleiteranschlusskabel 30 zu stecken ist. Die Instrumententestanordnung 18 umfasst eine oder mehrere Zündtestelektroden 19, 20, die vorzugsweise über Impedanzbauelemente 23, 24 mit Leitern 21, 22 des Geräts 10 oder des Neutralleiterkabels 30 verbunden sind. Die Zündtestelektroden 19, 20 sind in soweit zugänglich, dass mit einer funktionsfähigen Sonde oder einem Instrument 11 ein Funke zu beiden Zündtestelektroden 19, 20 gleichzeitig hergestellt werden kann.

### Bezugszeichenliste:

- 10: Gerät
- 11: Instrument
- 12: Neutralelektrodenanordnung
- 13: erste Teilelektrode
- 14: zweite Teilelektrode
- 15: Neutralleiterkabel
- 16: Gerätestecker
- 17: Neutralbuchse / gesonderte Buchse 17a
- 18: Instrumententestanordnung
- 18a: Elektrodenanordnung
- 19: erste Zündtestelektrode
- 20: zweite Zündtestelektrode
- 21: erster Neutralleiter
- 22: zweiter Neutralleiter
- 23: erstes Impedanzbauelement
- 24: zweites Impedanzbauelement
- 25: Splitschaltung
- 26: Isolatorkörper
- 27: Vertiefung
- 28: erster Kontakt
- 29: zweiter Kontakt
- 30: Neutralleiteranschlusskabel
- 31: Neutralelektrodenanschluss

## Patentansprüche

1. Instrumententestanordnung (18) insbesondere für medizinische Instrumente (11) zur Argon-Plasma-Koagulation,
mit einer Elektrodenanordnung (18a), die mindestens zwei Zündtestelektroden (19, 20) aufweist, die elektrisch mit einem Neutralleiter (21, 22) eines Neutralleiterkabels (15) oder eines Geräts (10) verbunden sind,
wobei das flexible Neutralleiterkabel (15) zwei Neutralleiter (21, 22) aufweist, die gegeneinander isoliert und an zwei Teilelektroden (13, 14) einer Neutralelektrodenanordnung (12) und an eine Splitschaltung (25) angeschlossen sind, die dazu dient, den ohmschen Widerstand oder die Impedanz zwischen den beiden Teilelektroden (13, 14) zu messen,
die Elektrodenanordnung (18a) zwei einander benachbart angeordnete Zündtestelektroden (19, 20) aufweist,
eine Zündtestelektrode (19) mit dem einen Neutralleiter (21) und die andere Zündtestelektrode (20) mit dem anderen Neutralleiter (22) verbunden ist und
die Zündelektrode (19, 20) über ein eine Impedanz aufweisendes Bauelement (23, 24) mit dem Neutralleiter (21, 22) verbunden ist.

2. Instrumententestanordnung nach Anspruch 1, **dadurch gekennzeichnet, dass** die Elektrodenanordnung (18a) Teil eines Gerätesteckers (16) ist, der mindestens einen Kontakt (29, 28) zum Anschluss an ein medizinisches Gerät (10) aufweist, so dass die Zündtestelektrode (19, 20) in oder an dem Gerätestecker (16) angeordnet ist, wobei sich von dem Gerätestecker (16) das flexible Neutralleiterkabel (15) weg zu einer Neutralelektrodenanordnung (12) oder einem Neutralelektrodenanschluss (31) hin erstreckt.

3. Instrumententestanordnung nach Anspruch 1, **dadurch gekennzeichnet, dass** die Elektrodenanordnung (18a) an dem flexiblen Neutralleiterkabel (15) zwischen einem Gerätestecker (16) und einer Neutralelektrodenanordnung (12) oder einem Neutralelektrodenanschluss (31) angeordnet ist.

4. Instrumententestanordnung nach Anspruch 1, **dadurch gekennzeichnet, dass** die Elektrodenanordnung (18a) an einem Neutralelektrodenanschluss (31) oder einer Neutralelektrodenanordnung (12) angeordnet ist, die über das flexible Neutralleiterkabel (15) mit einem Gerätestecker (16) verbunden sind.

5. Instrumententestanordnung nach Anspruch 1, **dadurch gekennzeichnet, dass** sie in oder an dem Gerät (10) angeordnet ist, das der Speisung des Instruments (11) dient oder dass die Instrumententestanordnung (18) als Zwischenstecker ausgebildet ist, der zwischen eine Neutralbuchse (17) des Geräts (10) und einen Gerätestecker (16) des Neutralleiterkabels (15) anzuordnen ist.

6. Instrumententestanordnung nach Anspruch 2, **dadurch gekennzeichnet, dass** der Gerätestecker (16) einen aus einem Isolatormaterial ausgebildeten Körper (26) aufweist, der die Zündtestelektrode (19, 20) trägt.

7. Instrumententestanordnung nach Anspruch 6, **dadurch gekennzeichnet, dass** der Gerätestecker (16) einen aus einem Isolatormaterial ausgebildeten Steckerkörper aufweist.

8. Instrumententestanordnung nach Anspruch 1, **dadurch gekennzeichnet, dass** das Bauelement (23, 24) eine Kapazität aufweist.

9. Instrumententestanordnung nach Anspruch 1, **dadurch gekennzeichnet, dass** das Bauelement (23, 24) ein ohmscher Widerstand ist.

10. Instrumententestanordnung nach den Ansprüchen 1 und einem der Ansprüche 11 bis 13, **dadurch gekennzeichnet, dass** jede Zündtestelektrode (19, 20) über das Bauelement (23, 24) mit ihrem jeweiligen Neutralleiter (21, 22) verbunden ist.

11. Instrumententestanordnung nach Anspruch 10, **dadurch gekennzeichnet, dass** die beiden Bauelemente (23, 24) gleiche elektrische Kennwerte aufweisen.

## Claims

1. Instrument test assembly (18), in particular for medical instruments (11) for argonplasma coagulation,
with an electrode arrangement (18a) which has at least two ignition test electrodes (19, 20) which are electrically connected to a neutral conductor (21, 22) of a neutral conductor cable (15) or of a device (10),
wherein the flexible neutral conductor cable (15) has two neutral conductors (21, 22) which are isolated from each other and connected to two part electrodes (13, 14) of a neutral electrode arrangement (12) and to a split circuit (25) which serves to measure the ohmic resistance or impedance between the two part electrodes (13, 14),
the electrode arrangement (18a) has two ignition test electrodes (19, 20) arranged adjacent to each other,
one ignition test electrode (19) is connected to the one neutral conductor (21) and the other ignition test electrode (20) is connected to the other neutral conductor (22), and the ignition electrode (19, 20) is connected to the neutral conductor (21, 22) via a component (23, 24) having an impedance.

2. Instrument test assembly according to claim 1, **characterised in that** the electrode arrangement (18a) is part of a device plug (16) which has at least one contact (29, 28) for connection to a medical device (10), so that the ignition test electrode (19, 20) is arranged in or on the device plug (16), wherein the flexible neutral conductor cable (15) extends from the device plug (16) to a neutral electrode arrangement (12) or a neutral electrode connection (31).

3. Instrument test assembly according to claim 1, **characterised in that** the electrode arrangement (18a) is arranged on the flexible neutral conductor cable (15) between a device plug (16) and a neutral electrode arrangement (12) or a neutral electrode connection (31).

4. Instrument test assembly according to claim 1, **characterised in that** the electrode arrangement (18a) is arranged on a neutral electrode connection (31) or a neutral electrode arrangement (12) which is connected to a device plug (16) via the flexible neutral conductor cable (15).

5. Instrument test assembly according to claim 1, **characterised in that** it is arranged in or on the device (10) which serves to supply the instrument (11), or that the instrument test assembly (18) is configured as an adapter plug which is to be arranged between a neutral socket (17) of the device (10) and a device plug (16) of the neutral conductor cable (15).

6. Instrument test assembly according to claim 2, **characterised in that** the device plug (16) has a body (26) which is formed from an isolating material and carries the ignition test electrode (19, 20).

7. Instrument test assembly according to claim 6, **characterised in that** the device plug (16) has a plug body formed from an isolating material.

8. Instrument test assembly according to claim 1, **characterised in that** the component (23, 24) has a capacitance.

9. Instrument test assembly according to claim 1, **characterised in that** the component (23, 24) is an ohmic resistor.

10. Instrument test assembly according to claim 1 and any of claims 11 to 13, **characterised in that** each ignition test electrode (19, 20 is connected to its respective neutral conductor (21, 22) via the component (23, 24).

11. Instrument test assembly according to claim 10, **characterised in that** the two components (23, 24) have the same electrical key values.

## Revendications

1. Agencement de test d'instruments (18), en particulier pour des instruments médicaux (11) destinés à la coagulation au plasma d'argon,
comprenant un agencement d'électrodes (18a) qui présente au moins deux électrodes d'essai d'allumage (19, 20) qui sont reliées électriquement à un conducteur neutre (21, 22) d'un câble de conducteur neutre (15) ou d'un appareil (10),
sachant que le câble de conducteurs neutres (15) souple présente deux conducteurs neutres (21, 22) qui sont isolés l'un vis-à-vis de l'autre et sont raccordés à deux électrodes élémentaires (13, 14) d'un agencement d'électrodes neutres (12) et à un circuit diviseur (25) qui sert à mesurer la résistance ohmique ou l'impédance entre les deux électrodes élémentaires (13, 14),
l'agencement d'électrodes (18a) présente deux électrodes d'essai d'allumage (19, 20) disposées à proximité l'une de l'autre,
une électrode d'essai d'allumage (19) est reliée à l'un (21) des conducteurs neutres et l'autre électrode d'essai d'allumage (20) est reliée à l'autre conducteur neutre (22), et
l'électrode d'allumage (19, 20) est reliée au conducteur neutre (21, 22) par l'intermédiaire d'un composant (23, 24) présentant une impédance.

2. Agencement de test d'instruments selon la revendication 1, **caractérisé en ce que** l'agencement d'électrodes (18a) fait partie d'un connecteur d'appareil (16) qui présente au moins un contact (29, 28) destiné au raccordement à un appareil médical (10), de sorte que l'électrode d'essai d'allumage (19, 20) est disposée dans ou sur le connecteur d'appareil (16), le câble de conducteurs neutres (15) souple s'étendant à partir du connecteur d'appareil (16), en direction d'un agencement d'électrodes neutres (12) ou d'une borne d'électrodes neutres (31).

3. Agencement de test d'instruments selon la revendication 1, **caractérisé en ce que** l'agencement d'électrodes (18a) est disposé sur le câble de conducteurs neutres (15) souple, entre un connecteur d'appareil (16) et un agencement d'électrodes neutres (12) ou une borne d'électrodes neutres (31).

4. Agencement de test d'instruments selon la revendication 1, **caractérisé en ce que** l'agencement d'électrodes (18a) est disposé sur une borne d'électrodes neutres (31) ou un agencement d'électrodes neutres (12) qui sont reliés à un connecteur d'appareil (16) par l'intermédiaire du câble de conducteurs neutres (15) souple.

5. Agencement de test d'instruments selon la revendication 1, **caractérisé en ce qu'**il est disposé dans ou sur l'appareil (10) qui sert à l'alimentation de l'instrument (11), ou **en ce que** l'agencement d'essai d'instruments (18) est réalisé sous forme de connecteur intermédiaire qui doit être disposé entre un connecteur femelle neutre (17) de l'appareil (10) et un connecteur d'appareil (16) du câble de conducteur neutre (15).

6. Agencement de test d'instruments selon la revendication 2, **caractérisé en ce que** le connecteur d'appareil (16) présente un corps (26) qui est réalisé à partir d'un matériau isolant et porte l'électrode d'essai d'allumage (19, 20).

7. Agencement de test d'instruments selon la revendication 6, **caractérisé en ce que** le connecteur d'appareil (16) présente un corps de connecteur réalisé à partir d'un matériau isolant.

8. Agencement de test d'instruments selon la revendication 1, **caractérisé en ce que** le composant (23, 24) présente une capacité.

9. Agencement de test d'instruments selon la revendication 1, **caractérisé en ce que** le composant (23, 24) est une résistance ohmique.

10. Agencement de test d'instruments selon la revendication 1 et l'une des revendications 11 à 13, **caractérisé en ce que** chaque électrode d'essai d'allumage (19, 20) est reliée à son conducteur neutre (21, 22) respectif, par l'intermédiaire du composant (23, 24).

11. Agencement de test d'instruments selon la revendication 10, **caractérisé en ce que** les deux composants (23, 24) présentent des caractéristiques électriques identiques.
